Europäisches Patentamt

European Patent Office

Office européen des brevets

(19)

(11) Publication number: **0 246 101**

**A2**

(12) **EUROPEAN PATENT APPLICATION**

(21) Application number: **87304300.4**

(22) Date of filing: **14.05.87**

(51) Int. Cl.³: **C 12 N 15/00**
C 07 K 15/04, C 12 P 21/02
//A61K39/21, G01N33/569

(30) Priority: **14.05.86 JP 108539/86**
**14.05.86 JP 108540/86**

(43) Date of publication of application:
**19.11.87 Bulletin 87/47**

(84) Designated Contracting States:
**CH DE FR GB IT LI NL**

(71) Applicant: **FUJIREBIO KABUSHIKI KAISHA also trading as FUJIREBIO INC.**
**6-7, Shimoochiai 4-chome Shinjuku-ku**
**Tokyo 161(JP)**

(71) Applicant: **Hatanaka, Masakazu**
**1-23, Takanohigashihiraki-cho Sakyo-ku**
**Kyoto-shi Kyoto 606(JP)**

(72) Inventor: **Hatanaka, Masakazu**
**1-23, Takanohigashihiraki-cho Sakyo-ku**
**Kyoto-shi Kyoto 606(JP)**

(72) Inventor: **Itamura, Shigeyuki Tsunashimahigashi Mansion**
**11-45, Tsunashimahigashi 3-chome**
**Kohoku-ku Yokohama-shi Kanagawa 223(JP)**

(72) Inventor: **Kitajima, Takashi**
**478, Kumakawa**
**Fussa shi Tokyo 197(JP)**

(74) Representative: **Silverman, Warren et al,**
**HASELTINE LAKE & CO. Hazlitt House 28 Southampton Buildings Chancery Lane**
**London WC2A 1AT(GB)**

(54) **Protein having HTLV-1 antigenic activity and preparation thereof.**

(57) Proteins are disclosed which react with the serum from an ATL (adult T-cell leukemia) patient but do not react with the serum from a healthy person. One type of protein contains pX-IV env gene products and it also reacts with anti p40$^x$ monoclonal antibody. Its molecular weight is 59K. The other protein contains gag, pX-IV and env gene products and it also reacts with anti p19 and p28 monoclonal antibody in addition to anti p40$^x$ monoclonal antibody. Its molecular weight is 100K. The production of the former protein utilises a plasmid constructed with lac promoter region, synthetic codon, and env and pX-IV gene regions, and the production of the latter protein utilises a plasmid having lac promoter region, synthetic codon, and gag, env, and pX-IV gene regions. These proteins are useful for detection of the antibodies of ATLA.

## PROTEIN HAVING HTLV-I ANTIGENIC ACTIVITY AND PREPARATION THEREOF

This invention relates to protein materials having HTLV-I antigenic activity and to the production thereof. More particularly, the present invention relates to novel antigenic proteins to react with antibodies to HTLV-I antigens and to methods of producing the same wherein novel plasmids are utilised.

Recently, it has been reported that human T-cell leukemia virus-I (HTLV-I) and adult T-cell leukemia virus (ATLV) are homologous retroviruses. Since natural antibodies to HTLV-I antigens are produced in sera from patients infected with HTLV-I, infection with HTLV-I can be found by detecting these antibodies in sera. This would require the provision of appropriate antigens.

It is known that a group of adult T-cell leukemia-associated antigens (ATLA) consisting of p70, gp68, p53, gp46, $p40^X$, p36, p28, p24, p21, p19, p15 and p14 exist in adult T-cell leukemia (ATL) cell lines (Yamamoto, N. and Hinuma, Y., Int. J. Cancer, 30, 289-293 (1982)). It is also known that both ATL patients and HTLV-I patients have antibodies specific to ATLA (Hinuma, Y. et al. Proc. Natl. Acad. Sci. U.S.A., 78, 6476-6480 (1982)).

On the other hand, various methods of detecting the antibodies to HTLV-I or ATLV have been developed, although the antigens utilised are usually HTLV-I- or ATLV-producing cells. A technique for separation of viral antigens of these viruses from the culture supernatant of these cells has also been developed (Japanese Patent KOKAI No.58-187861). The inventors there have succeeded in isolating antigenic protein recognised by monoclonal antibodies to p19 and p24 which are gag gene product of HTLV-I (Itamura et al., Gene, 38, 57-64 (1985)).

However, when using HTLV-I or ATLV-producing cells themselves as antigen, a non-specific reaction occurs, and therefore accuracy of the result obtained is a problem. Moreover, when using such viral antigen, there is the problem that the amount of antigen obtainable from culture supernatant is small.

In contrast, it has been found that when ATLV antigen is produced by a more direct method, it may be produced in substantial quantity notwithstanding that a simple procedure is adopted. Besides, when an antigenic protein corresponding to a gene region different from that of the prior antigenic protein is obtained, detection of the antibodies to ATLA can be made more certain by combining this novel antigenic protein with the prior antigenic protein. Many ways of analysing the antibodies become possible.

Thus in one aspect this invention provides a protein containing pX-IV and env gene products, which protein reacts with the serum from an ATL patient and with anti p40$^X$ monoclonal antibody, does not react with the serum from a healthy person, and has a molecular weight, determined by electrophoresis, of 59K.

In a second aspect, this invention provides a protein containing gag, pX-IV and env gene products, which protein reacts with the serum from an ATL patient, and with anti p19 and p28 monoclonal antibody and anti p40$^X$ monoclonal antibody, does not react with the serum from a healthy person, and has a molecular weight, determined by electrophoresis, of 100K.

The proteins of this invention are based on expression plasmids of env-pX-IV complex gene product and gag-env-pX-IV complex gene product respectively of HTLV-I, and are able to react with the antibody to p40$^X$ or to p19, p28 and p40$^X$ antibodies respectively of ATLV antigens. Thus the proteins of this invention may be produced by culturing a microorganism harbouring one or other of the above expression plasmids.

Thus, the present invention may be summarised as relating to antigenic proteins reacting with the antibody

to the p40$^X$ antibody to the group of ATLV antigens and to the preparation thereof.

In the following description, reference will be made to the accompanying drawings wherein:

FIGURES 1 and 2 are diagrams illustrating routes for constructing plasmids embodying this invention; and

FIGURES 3 and 4 are electrophoretic migration patterns of the serum from an ATL patient, the serum from a healthy person and monoclonal antibodies to specific ATLA.

Antigenic protein reacting with the antibody to p40$^X$ of the ATLV antigens group is produced by culturing a microorganism harbouring the plasmid pKI-3 having lac promoter region, synthetic codon and env-pX-IV gene region of HTLV-I.

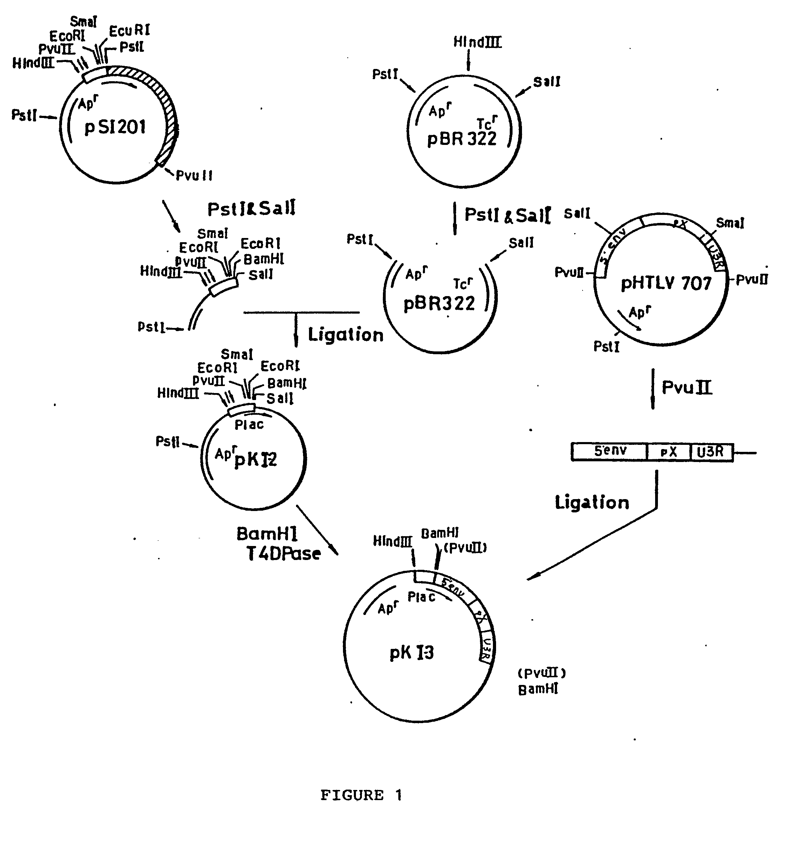

In one method of constructing the plasmid pKI-3, expression vector pSI201 having a lac promoter region and SD sequence and start codon necessary for translation is first constructed. This vector pSI201 is digested with SalI and PstI to produce a SalI-PstI fragment. Meanwhile pBR322 is similarly treated with restriction enzymes to produce a SalI-PstI fragment, and ligated with the foregoing SalI-PstI fragment of PSI201 to obtain plasmid pKI-2. In order to ligate the env-pX-IV gene of HTLV-I onto plasmid pKI-2, plasmid pKI-2 is treated with BamHI. Meanwhile, plasmid pHTLV707 containing the env-pX-U3R gene region of HTLV-I is treated with PvuII to produce an env-pX-U3R fragment which is then ligated with the above plasmid pKI-2 to obtain plasmid pKI-3. This method of construction is illustrated in Figure 1 of the accompanying drawings. As can be seen from this drawing, plasmid pKI-3 comprises a lac promoter region and the env-pX gene region of HTLV-I.

Plasmid pKI-3 may be introduced into a microorganism by using any suitable method for introducing a plasmid or a vector into a microorganism. The microorganism may be one of the commonly used

microorganisms such as <u>Escherichia</u> <u>coli</u>, <u>Bacillus</u> <u>subtilis</u> or <u>Pseudomonas</u> <u>aeruginosa</u>.

Cultivation of the microorganism harbouring plasmid pKI-3 may also employ conventional techniques. The microorganism harbouring the plasmid is selectively multiplied by adding ampicillin or the like. During cultivation, env-pX-IV complex gene product is induced by the addition of for example isopropyl-β-D-thiogalacto-pyranoside or adenosine-3',5'-cyclic monophosphate. When the amount of the antigenic protein reaches a maximum accumulation, the cultivation is stopped. Since the antigenic protein is accumulated in the cells, the multiplied cells are ruptured by, for example, enzyme treatment, freezing and thawing, or ultrasonication. After the rupturing, the supernatant is recovered by centrifugation, and pure antigenic protein can be obtained from the supernatant by affinity chromatography using an anti-ATLA antibody or by electrophoresis.

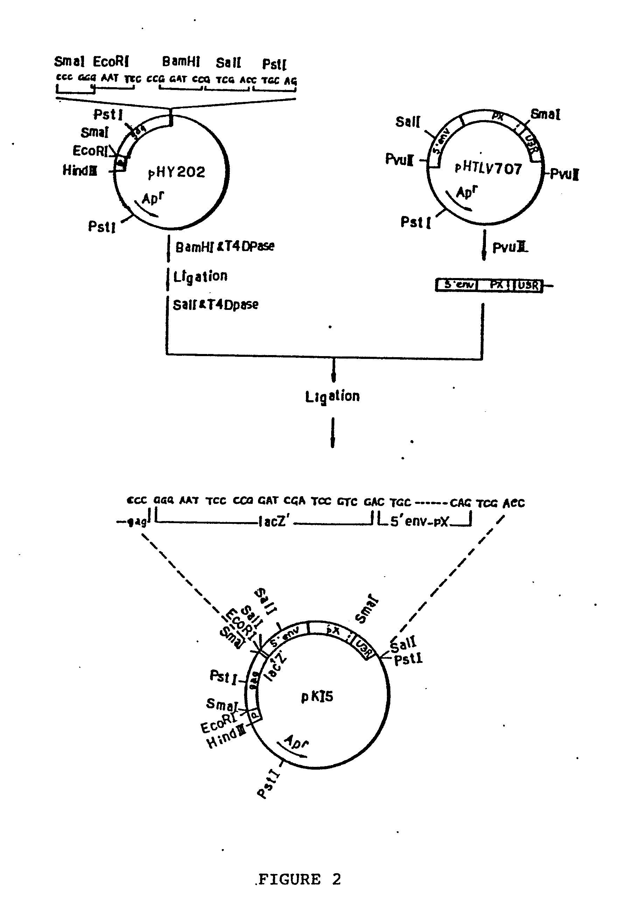

A related protein which reacts with antibodies to HTLV-I antigens is an antigenic protein which reacts with the antibody to the p19, p28 and $p40^X$ group of ATLV antigens. This is produced by culturing a microorganism harbouring the plasmid pKI-5 instead of the plasmid pKI-3. The plasmid pKI-5 is composed of lac promoter region, gag gene region and env-pX gene region of HTLV-I as shown in Figure 2 of the accompanying drawings.

In one method of constructing the plasmid pKI-5, expression vector pSI201 is first constructed by introducing a synthetic linker having smaI region into the EcoRI region existing downstream of the region of the start codon of expression vector pSI101 having lac promoter region and SD sequence and start codon necessary for translation. Subsequently, the gag gene region is cut off from HTLV-I provirus by smaI, and ligated at the smaI site of vector pSI201 to obtain plasmid pHY202. The plasmid pHY202 is treated with BamHI and SalI in order to ligate it with the env-pX-IV gene of HTLV-I. With this aim in view, the env-pX-U3R gene will have been cut off from plasmid pHTLV707 having env-pX-U3R gene of HTLV-I as part of PvuII, and ligated with the above plasmid pHY202 to obtain plasmid pKI-5. This constructional method is illustrated in Figure 2. As shown in this drawing, plasmid pKI-5 is composed of lac promoter region, gag gene region of HTLV-I and env-pX gene region of HTLV-I.

The microorganism for harbouring plasmid pKI-5 and its introduction thereinto may be the same as mentioned in connection with plasmid pKI-3. Cultivation of the microorganism and separation of the antigenic protein may also be carried out using a procedure as described for plasmid pKI-3.

The antigenic protein produced by a microorganism harbouring plasmid pKI-3 reacts strongly with the serum from an ATL patient but does not react with the serum from a healthy person. This antigenic protein also reacts with anti p40$^X$ monoclonal antibody. The molecular weight of this protein is 59K as determined by electrophoresis, and this result is consistent with the molecular weight of the protein being about 60K calculated from the DNA sequence of Figure 1. Accordingly, this antigenic protein is a conjugated protein containing not only pX-IV gene product but also env gene product. Since a cell extract of MT-2, which is an established cell line derived from leucocytes, does not contain 59K antigenic protein, this antigenic protein is novel.

The antigenic protein produced by a microorganism harbouring plasmid pKI-5 also reacts strongly with the serum from an ATL patient but does not react with the serum from a healthy person. This antigenic protein reacts with the monoclonal antibody reacting with p19 and p28 of ATLA and the monoclonal antibody reacting with p40$^X$ of ATLA. The molecular weight of this protein is 100K as determined by electrophoresis and this result is consistent with the molecular weight of the protein being about 100K as calculated from the DNA sequence of Figure 2. Accordingly, this antigenic protein is a conjugated protein containing not only gag gene product and pX-IV gene product but also env gene product. Since a cell extract of MT-2 cell line does not contain 100K antigenic protein, this antigenic protein is also novel.

The following Examples illustrate this invention:

## EXAMPLE 1

(1) Construction of Plasmid pSI201

A synthetic oligonucleotide consisting of dAATTCCCGGG was introduced into the EcoRI region of PSI101 having a lac promoter region (Itamura et al., Gene, 38, 57-64 (1985)) to construct plasmid pSI201.

(2) Construction of Plasmid pKI-2

The thus constructed plasmid pSI201 was treated with restriction enzymes SalI and PstI to produce 1.1 Kb SalI-PstI fragment of pSI201. At the same time, pBR322 was similarly treated with the same restriction enzymes to produce 3.0 Kb SalI-PstI fragment of pBR322, and this was then ligated with the foregoing 1.1 Kb SalI-PstI fragment of pSI201 to construct plasmid pKI-2.

(3) Construction of Plasmid pKI-3

The PvuII fragment of plasmid pHTLV707 having the env-pX-IV-U3R gene region of the DNA provirus of HTLV-I (Takeuchi et al., J. Gen. Virol., 66, 1825-1829 (1985)) was cut off, and ligated at the SalI site of plasmid pKI-2 previously treated with BamHI to construct plasmid pKI-3.

(4) Introduction of pKI-3 into E. coli.

The thus constructed plasmid pKI-3 was introduced into Escherichia coli K12 strain 545π (Bakkari, A.I., and Zipser, D., Nature New Biol., 243, 238-241 (1973)) by the calcium chloride method.

(5) Production of Antigenic Protein

The E. coli cells thus harbouring pKI-3 were cultured at 32°C to 80 Klett units in L-broth containing 100 µg/ml ampicillin. In order to induce an env-pX-IV gene product, isopropyl- β -D-thiogalactopyranoside and adenosine-3',5'-cyclic monophosphate, each in amounts of 1 mM, were added to the broth, and further culturing took place at $32^{O}$C to 100 Klett units. Cells were collected by centrifugation, and suspended in a concentration of 1 x $10^{10}$ cells/ml in a solution containing 10 mM Tris-HCl (pH 8.0), 1 mM EDTA, 0.02% by weight $NaN_3$ and 10 µg/ml phenylmethylsulphonyl fluoride (PMSF). This cell suspension was digested with 1 mg/ml lysozyme at $0^{O}$ for 10 minutes, and freezing and thawing were repeated three times. 20 µg/ml DNase I were added to the suspension which was digested at $0^{O}$C for 10 minutes. Then, the supernatant was recovered by centrifugation, and used as cell extract.

For comparison purposes, the plasmid pKI-2 was introduced into E. coli K12 545π, and treated in the same manner as above to obtain a supernatant.

(6) Separation of Antigenic Protein and Its Detection

The cell extracts thus obtained in amounts corresponding to 1 x $10^7$ cells and an extract of MT-2 cells in an amount corresponding to 2 x $10^4$ cells which had been separately prepared were subjected to electrophoresis, with separation of their protein onto 12% sodium dodecyl sulphate-polyacrylamide gel taking place. Then, the proteins in the gel were transferred to a nitrocellulose membrane by electrophoretic migration, and allowed to react with the serum (A) from an ATL patient (antibody titre: 640 times), a monoclonal antibody recognising $p40^X$ (B) and the serum (C) from a healthy adult. The immune complex thereby formed was detected by enzyme immunoassay using peroxidase-bound protein A (E.Y. Laboratories, Inc.) (A, C) or peroxidase-bound anti mouse IgG (Cappel Laboratories, Inc.) (B).

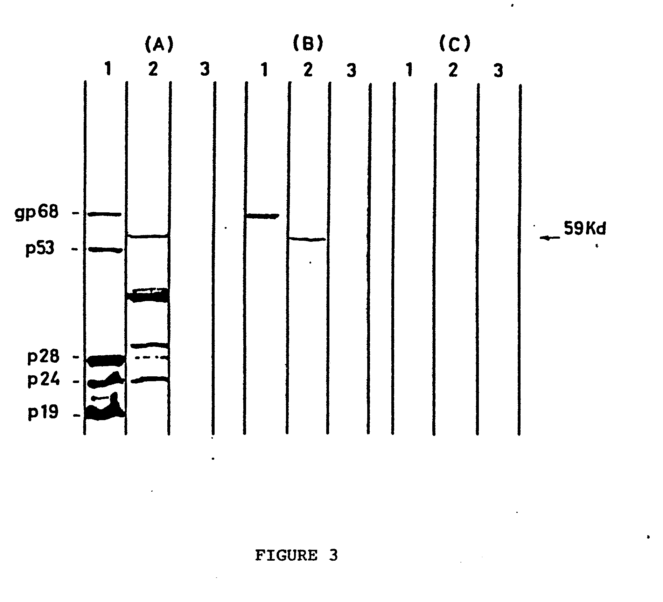

The results are shown in Figure 3 of the accompanying drawings, the first column of each group being for the MT-2 cell extract, the second column being for the extract of E. coli containing the antigenic protein of the invention, and the third column being for the comparative extract of E. coli containing pKI-2. As a criterion of molecular weight, there was used a standard solution (Bethesda Research Laboratories, Inc.) containing myosin (200K), phosphorylase B (92.5K), bovine serum albumin (68K) and egg albumin (43K).

EXAMPLE 2

(1) Construction of Plasmid pHY202

Use was to be made of plasmid pHY202 which had been developed by the inventors (Itamura et al., Gene, 38, 57-64 (1985)). To produce it, a synthetic oligonucleotide consisting of dAATTCCCGGG was introduced into the EcoRI region of plasmid pSI101 having a lac promoter region to construct plasmid pSI201. Subsequently, a 1.1 Kb SmaI fragment of the gag gene of HTLV-I was ligated with the smaI region of pSI201 to construct plasmid pHY202.

(2) Construction of Plasmid pKI-5

A PvuII fragment of plasmid pHTLV707 containing the env-pX-IV-U3R gene region of the DNA provirus of HTLV-I was cut off, and ligated at the salI site of plasmid pHY-2 which had previously been treated with BamHI to construct plasmid pKI-5.

(3) Introduction of pKI-5 into E. coli.

The thus constructed plasmid pKI-5 was introduced into Escherichia coli K12 strain 545 π by the calcium chloride method.

(4) Production of Antigenic Protein

The E. coli cells thus harbouring pKI-5 were cultured in the same manner as the modified cells in step (5) of Example 1 and the culture product was worked up in the same manner as specified by step (5) of Example 1 to yield a cell extract.

For comparison purposes pSI201 was introduced into E. coli K12 545 , and treated in the same manner as above to obtain a supernatant.

(5) Separation of Antigenic Protein and Its Detection

The cell extracts thus obtained in amounts corresponding to 1 x $10^7$ cells and an extract of MT-2 cells in an amount corresponding to 2 x $10^4$ cells which had been separately prepared were subjected to electrophoresis, with separation of their protein onto 12% sodium dodecyl sulphate-polyacrylamide gel taking place. Then, the proteins in the gel were transferred to a nitrocellulose membrane by electrophoretic migration, and allowed to react with a monoclonal antibody FR19/28 recognising p19 and p28 (D), the serum (E) from an ATL patient (antibody titre: 640 times), a monoclonal antibody recognising $p40^X$ (F), and the serum from a healthy adult (G). The immune complex thereby formed in each case was detected by the enzyme immunoassay method using peroxidase-bound protein A (E.Y. Laboratories, Inc.) (E, G) or peroxidase-bound anti mouse IgG (Cappel Laboratories, Inc.) (D, F).

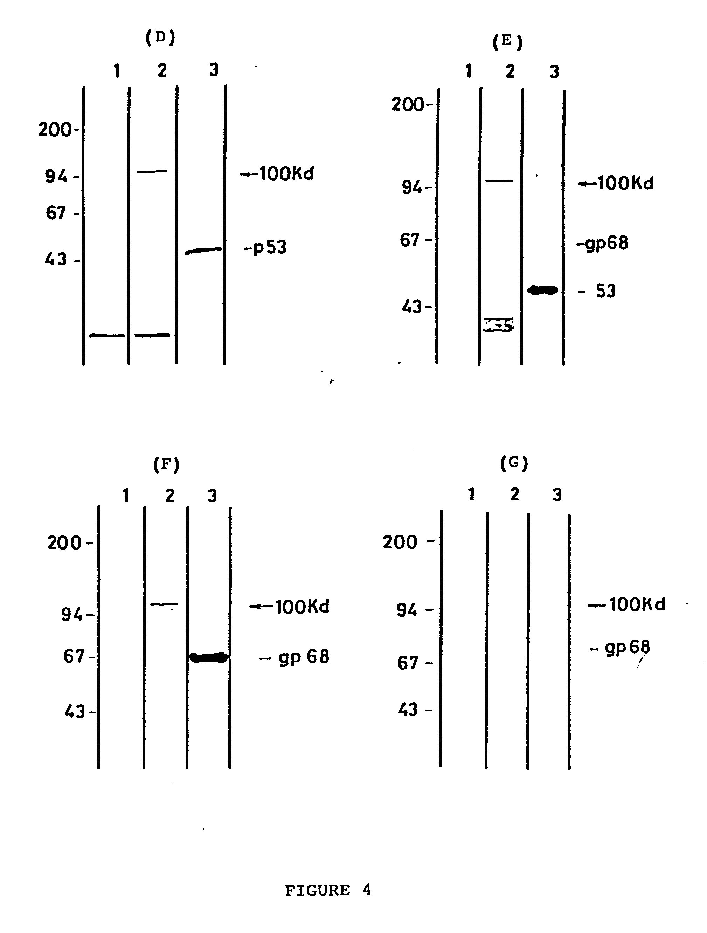

The results are shown in Figure 4 of the accompanying drawings. In Figure 4, the first column of each group is for the extract of comparative E. coli having pSI201, the second column is for the extract of E. coli containing the antigenic protein of the invention, and the third column is for the MT-2 cell extract. The same standard solution as in Example 1 was employed as a molecular weight criterion.

Claims:

1. A protein containing pX-IV and env gene products, which protein reacts with the serum from an ATL patient and with anti p40$^X$ monoclonal antibody, does not react with the serum from a healthy person, and has a molecular weight, determined by electrophoresis, of 59K.

2. A protein containing gag, pX-IV and env gene products, which protein reacts with the serum from an ATL patient, and with anti p19 and p28 monoclonal antibody and anti p40$^X$ monoclonal antibody, does not react with the serum from a healthy person, and has a molecular weight, determined by electrophoresis, of 100K.

3. A method of preparing a protein according to claim 1, which comprises culturing a microorganism harbouring plasmid having lac promotor region, synthetic codon, and env and pX-IV gene regions, and separating said protein from the cultured mixture.

4. The method of claim 3, wherein said plasmid is plasmid pKI-3.

5. A method of preparing a protein according to claim 4, which comprises culturing a microorganism harbouring plasmid having lac promoter region, synthetic codon, and gag, env and pX-IV gene regions, and separating said protein from the cultured mixture.

6. The method of claim 5, wherein said plasmid is plasmid pKI-5.

7. The method of any one of claims 3 to 6, wherein the microorganism is Escherichia coli, Bacillus subtilis or Pseudomonas aeruginosa.

8. The method of any one of claims 3 to 7, wherein the recovery of the protein produced includes rupture of multiplied cells of said microorganism.

9. The method of claim 8, wherein the protein is recovered from the resulting supernatant by affinity chromatography using an anti-ATLA antibody.

10. The method of claim 8, wherein the protein is recovered from the resulting supernatant by electrophoresis.

FIGURE 1

214

FIGURE 2

FIGURE 3

FIGURE 4